Europäisches Patentamt

European Patent Office

Office européen des brevets

(19)

(11) Publication number: **0 133 326**
A2

# EUROPEAN PATENT APPLICATION

(21) Application number: **84200254.5**

(22) Date of filing: **22.02.84**

(51) Int. Cl.⁴: **G 01 N 33/497**, G 01 N 31/22

(30) Priority: **27.06.83 US 508108**

(43) Date of publication of application: **20.02.85**
**Bulletin 85/8**

(84) Designated Contracting States: **AT BE CH DE FR GB IT LI LU NL SE**

(71) Applicant: **Diversified Medical Technologies, Inc., Suite 115 6200 South Syracuse Street, Inglewood Colorado 80202 (US)**

(72) Inventor: **Schmitz II, Robert E., 88 East Belmeadow Lane, Chagrin Falls Ohio 44022 (US)**

(74) Representative: **de Boer, Hindrik Geert Jan et al, de Lairessestraat 131-135, NL-1075 HJ Amsterdam (NL)**

(54) **Disposable breath analyzer.**

(57) A portable, pocket-size, disposable breath analyzer device (10) is disclosed characterized by a tubular housing (12) having an end portion (14) formed to be placed in the mouth of a user and a central portion carrying a canister (24) filled with a quantity of potassium dichromate impregnated silica gel (30) adapted to provide a visual color indication of the amount of alcohol contained in a user's breath sample blown through the housing. A pair of valve members (40) are positioned on opposite sides of the canister within the housing to prevent any back aspiration of air to the user from the canister (24). Three embodiments of the invention are disclosed, each adapted to yield a visual indication of the approximate blood alcohol level of an individual user.

# DISPOSABLE BREATH ANALYZER

## BACKGROUND OF THE INVENTION

The present invention relates generally to devices for measuring alcohol content of gases and more particularly to a portable, pocket-size, disposable breath analyzer device for determining the blood alcohol content of a user.

In recent years, the number of vehicular deaths attributed to intoxication of drivers has dramatically increased. In response to this increase, the public has demanded more stringent legislation and enforcement procedures against intoxicated drivers. Due to the inherent inaccuracy of visual observation determinations of the intoxication of individuals, most legislation has attempted to define intoxication by use of the blood alcohol concentration of individuals with values of 0.1 through 0.2 percent alcohol/blood concentration generally being the critical range for legal intoxication. Due to the intoxication standards being defined in terms of the blood alcohol concentration, there is a need for individuals as well as law enforcement personnel to have means for approximately determining blood alcohol concentration levels.

There are two prior art methods which are generally utilized to determine the blood alcohol concentration level of an individual; the first being actual blood sample analysis and the second being breath sample analysis. Although both of these prior art methods have proven generally effective for their intended purpose, they each possess inherent deficiencies which have detracted from their overall effectiveness and widespread use by the public as well as law enforcement agencies.

As is well known, blood sample analysis, although being highly accurate, requires an invasion or intrusion into the body of an individual which raises concerns for use by law enforcement personnel. In addition, once a blood sample has been obtained from an individual, actual analysis of a blood sample is generally accomplished through professionally trained laboratory technicians. As such, blood sample analysis or tests have proven inapplicable for use by drinking individuals to self-ascertain their precise alcohol blood levels or by typical law enforcement personnel. Further, the blood sample analysis has proven deficient in that it typically cannot be utilized directly in the field, thereby allowing the actual alcohol concentration in the blood to be reduced through the normal metabolic process of an individual during transport to a suitable blood sample analysis laboratory.

The prior art breath sample analysis correlates the amount of alcohol in a user's breath with the amount of alcohol in the same person's blood. Although such breath analysis does not require an actual invasion into the user's body, it is still frought with the inability to provide an on-the-scene determination of intoxication due to the analysis equipment of such breath samples heretofore comprising extremely costly and complicated laboratory apparatus. Although these deficiencies have been recognized to a limited extent in the prior art, the proposed solutions to date have either been extremely expensive, unreliable or have posed a health hazard to the individual users.

As such, there exists a substantial need in the art for means of allowing both an individual user and law enforcement officer to make reliable, low-cost, non-invasive determinations of blood alcohol concentration levels directly in the field.

## SUMMARY OF THE INVENTION

The present invention specifically addresses and alleviates the above-referenced deficiencies associated in the art by providing a portable, pocket-size and disposable breath analyzer device.

More particularly, the present invention discloses three embodiments of a disposable breath analyzer device all characterized by the use of a tubular housing having an end portion formed to be placed in the mouth of a user, and a central portion carrying a canister filled with a quantity of colorimetric chemical material such as potassium dichromate impregnated silica gel. The particular silica gel compound is adapted to provide a visual color indication of the amount of alcohol contained in a user's breath sample blown through the housing. By suitable construction of the housing and canister, the linear color change of the silica gel compound can be calibrated to provide a direct visual indication of the percent blood alcohol concentration on the breath sample . As such, the present invention permits a rapid, in-the-field determination by a user or law enforcement officer of the blood alcohol concentration level of an individual.

To insure the safety of the individual user, the present invention additionally incorporates a pair of valve members positioned on opposite sides of the canister within the housing which prevent any back aspiration of air from the silica gel compound to the user. In addition, when the present invention is utilized in conjunction with an expansible bag sized to insure a consistent breath sample volume being blown to the housing, these valving members prevent any return of air contained in the expansible bag back to the canister.

In the first embodiment of the invention, the housing is calibrated along a linear scale to provide a visual determination of the actual blood alcohol concentration level of the user. By introduction of a suitable buffer such as bisodium bisulfate to the potassium dichromate impregnated silica gel, the effective range of the calibration scale may be adjusted to desired limits; for instance, from 0.05 through 0.3 percent alcohol blood concentration levels.

In a second embodiment of the invention, the housing may include a manifold which diverts the breath sample into a plurality of discrete channels, each of which is in communication with a separate chemically filled canister. The canisters are preferably calibrated to cause a color change at precise blood alcohol concentration levels, for instance, 0.05, 0.10, 0.15, and 0.20 percent. Thus, by noting which particular canister has changed color within the device and which have not, a user can obtain a rapid determination of his particular alcohol concentration level.

In a third embodiment of the invention, a novel piston arrangement is provided within the interior of the housing which is adapted to insure that only a particular breath sample volume is blown through the device. Thus, the accuracy of the device which is, of course, dependent upon the volume of breath sample being blown through the canister is insured.

BRIEF DESCRIPTION OF THE DRAWINGS

These as well as other features of the present invention will become more apparent upon reference to the drawings, wherein:

Figure 1 is a perspective view of a first embodiment of the disposable breath analyzer of the present invention;

Figure 2 is a cross-sectional view through the housing of the first embodiment of the present invention illustrating the internal components thereof;

Figure 3 is a perspective view of the canister of the present invention filled with a colorimetric chemical compound;

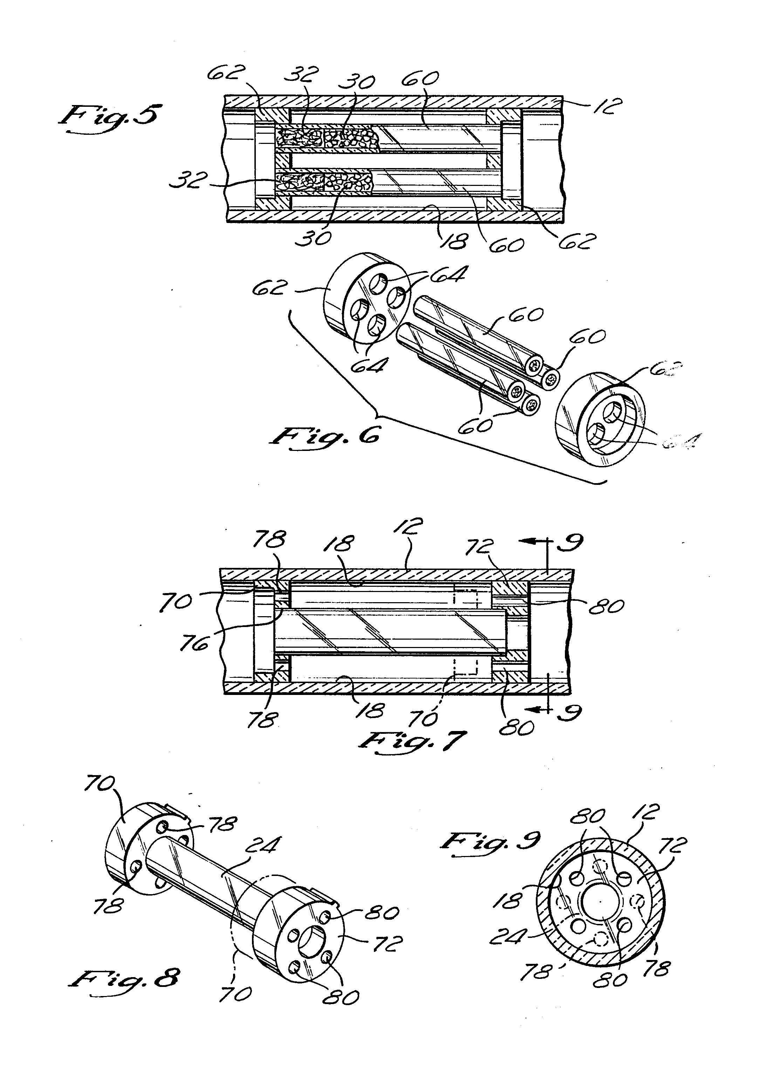

Figure 4 is a perspective view illustrating the manner in which an expansible bag may be positioned over the end of the breath analyzer of the present invention to insure that the proper volume of breath sample is blown through the housing;

Figure 5 is a cross-sectional view of the plural canisters utilized in the second embodiment of the present invention;

Figure 6 is an exploded view illustrating the manifold assembly utilized in the second embodiment of the present invention;

Figure 7 is a cross-sectional view of the third embodiment of the present invention illustrating its reciprocal mounted piston disposed within the housing;

Figure 8 is an enlarged perspective view depicting the manner in which the reciprocally mounted piston meters the amount of breath sample being blown through the housing; and

Figure 9 is a cross-sectional view taken about lines 9-9 of Figure 7.

DESCRIPTION OF THE PREFERRED EMBODIMENT

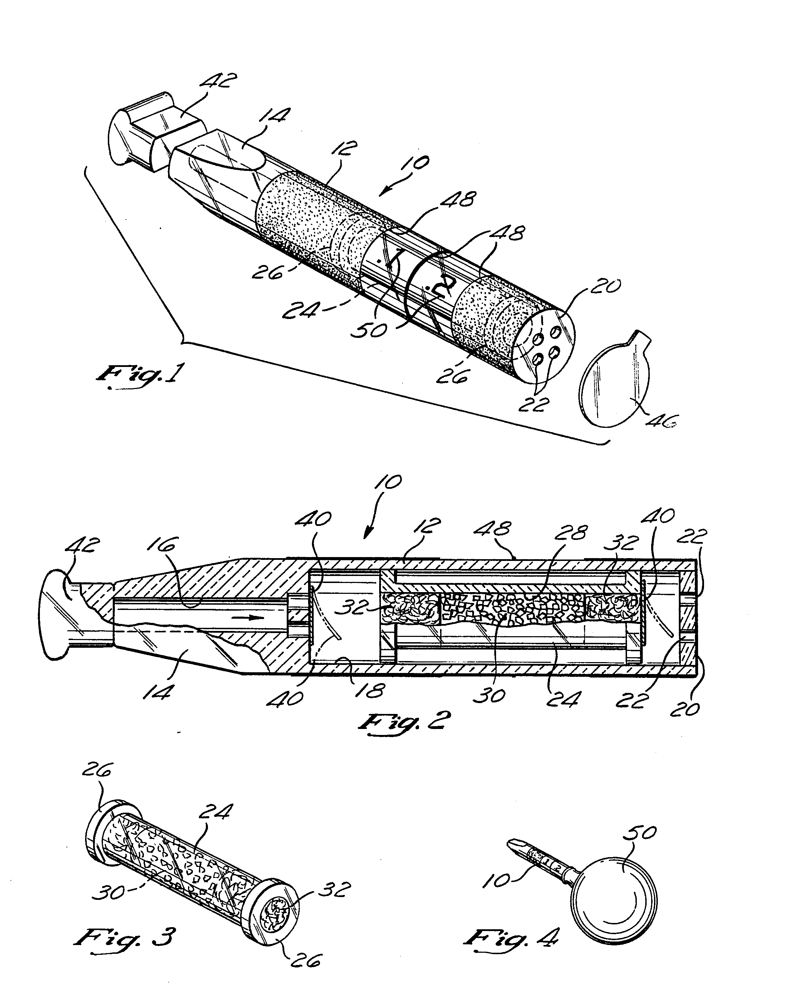

Referring to Figures 1 through 3, there is shown a first embodiment of the disposable breath analyzer device 10 of the present invention. The analyzer 10 is composed generally of an elongate tubular housing 12 preferably formed of a transparent plastic material. The housing 12 includes one end 14 having a tapered configuration adapted to form a mouthpiece which may be inserted into the mouth of a user (not shown). The mouth piece end 14 includes a central aperture 16 which extends axially through the housing 12 and communicates with an enlarged diameter chamber 18 formed within the housing 12. An end cap or plate 20 is additionally provided adjacent the opposite end of the chamber 18. One or more apertures 22 are provided in the end cap 20 such that air or, more particularly, breath from a user flow axially through the housing 12 in a direction indicated by the arrow in Figure 2.

A canister 24 is mounted within the interior of the chamber 18 preferably formed in a generally cylindrical configuration having a pair of end flanges 26 sized to have an outside diameter equal to the inside diameter of the chamber 18. The canister 24 is preferably formed of a transparent plastic material and additionally includes a central aperture 28 extending axially therethrough. A colorimetric chemical compound is disposed within the aperture 28 formed within the canister 24 and is maintained therein by a pair of mesh-like plugs 32 which although form a barrier to migration of the

chemical compound 30 within the aperture 28, permit the flow of air or breath therethrough.

As is well known, such colorimetric chemical compounds 30 are characterized by the ability to scrub or absorb alcohol carried by a gas and upon absorption of the same, experience a visual color change. Although a variety of such colorimetric compounds may be utilized, the preferred embodiment uses a potassium dichomate impregnated silica gel compound. This particular compound is characterized by a normally bright yellow color, which upon absorption of alcohol from a gas, changes to a brilliant green color. This chemical compound may additionally be buffered by use of a sodium bisulfate to modify the absorption rate of alcohol.

As best shown in Figure 2, one end of the chamber 18 and canister 24 is provided with a valving member 40 which in the preferred embodiment comprises a flapper type diaphragm or ball check valve which serves to permit gas flow through the housing 12 in only the direction indicated by the arrow in Figure 2. As will be recognized, both flapper valves 40 are biased in a normally planar configuration (indicated by the straight line positions of Figure 2) and selectively open to the phantom line position depicted in Figure 2. Hence, upon a breath sample being forced through the aperture 16 through the mouth piece end 14, the valving members 40 move to their phantom line position allowing the breath sample to enter into the chamber 18 and canister 24. Conversely, when the breath sample is discontinued, or upon the existence of a partial vacuum at the mouth piece, the valving members 40 return to their full line position and block any gas flow through the canister 24 and chamber 18. Hence, the valving members 40 provide a fail safe mechanism to prevent any back aspiration of air or chemical compound 30 to the user.

To insure that the chemical compound 30 does not react with water vapor or other gaseous compounds during storage, opposite ends of the housing 12 are preferably provided with removable seals 42 and 46.  The seal 42 may be secured by a suitable adhesive to the mouth piece end 14 so as to cover the aperture 16 while the seal 42 may be adhesively secured to the end plate 20 so as to cover the plural apertures 22.  When it is desired to utilize the analyzer 10, a user may mechanically break the adhesive bond between the end cap 42 from the mouthpiece 14 and strip the seal 42 off the end plate 22 such that the aperture 16 and apertures 22 are opened.  The housing 12 additionally may be provided with one or more calibration lines 48 which extend around its circumference and are in alignment with the chemical compound 30 retained within the central portion of the canister 24. In addition, suitable indicia 50 may be provided to indicate the particular calibration of the lines 48 in relation to the blood alcohol concentration level of the breath sample.

     With the structure defined, the operation of the analyzer 10 of the present invention may be described. As will be recognized, due to the relatively small size (i.e. approximately one-half inch in diameter by five inches in length) of the analyzer 10, it may easily be transported by a user or law enforcement agency.  When it is desired to obtain the blood alcohol concentration level of an individual, the end seals 42 and 46 may be removed in the manner previously described.  The user may subsequently insert the mouthpiece 14 within his mouth and exhale, causing a breath sample to enter into a aperture 16.

     Due to the pressure of the breath sample, the valving members 40 move from their full line position to their phantom line position to allow the breath sample to enter into and pass throught the cavity 18.  As the breath sample is forced through the aperture 28 of the canister 24, the alcohol contained within the sample is absorbed or scrubbed by the colorimetric compound 30, causing the compound 30 to change from its initial yellow color to a subsequent green color.  The scrubbing properties of the chemical compound 30 are such that the color change is linear and proportional to the amount of alcohol absorbed by the compound 30. As such, for a given volume of an air sample, a linear change in color of the component from left to right (as viewed in Figure 2) will be provided.

After the sample is completed, a user may merely inspect the housing 12 looking radially inward toward the canister 24 to note the axial length of the color change of the colorimetric component 30. The scale lines 48 thereby may be utilized to obtain an indication of the actual blood alcohol concentration of the individual. Upon completion of the viewing, the entire housing 12 may be discarded or alternatively maintained for future reference as by law enforcement personnel.

As will be recognized, the accuracy of the device 10 is limited primarily by the volume of the breath air sample being blown through the device. In most instances, suitable reliability can be obtained by exerting a moderate blow pressure into the mouthpiece 14 which is maintained for a specific time interval. However, when greater accuracy of the air sample is desired, a balloon 52 which is expansible to a maximum volume representative of the desired breath sample size may be mounted to the distal end of the housing 12. Due to the valving members 40 permitting flow through the housing 12 in only the direction indicated by the arrow in Figure 2, back aspiration of the breath sample from the balloon 52 is eliminated.

Referring to Figures 5 and 6, a second embodiment of the breath analyzer device 10 of the present invention is depicted. Construction of the second embodiment of the invention is identical to the first embodiment except that the canister 24 is replaced by a plurality of canisters 60 which are mounted in the chamber 18 by way of a pair of manifolds 62. As shown, each of the manifolds 62 includes a plurality of apertures 64 extending therethrough which receive one end of each of the canisters 60. The outside diameter of each of the manifolds 62 is additionally sized to be equal to the diameter of the chamber 18 such that the air sample blown through the chamber 18 is diverted through each of the apertures 62 and canisters 60.

The canisters 60 are provided with a quantity of potassium dichromate impregnated silica gel 30 which is maintained within each of the canisters 60 by a suitable mesh plug 32. However, in this second embodiment, the silica gel 30 is buffered with sodium bisulfate such that the scrubbing action of the silica gel 30 does not begin until a threshhold amount of alcohol contacts the silica gel 30. By proper buffering, the activation of each of the quantities of silica gel 30 within the plural canisters 60 can be incremented between suitable values, for instance, 0.05, 0.10, 0.15, and 0.20 percent blood alcohol. Hence, in use, when a breath sample is blown through the housing 12, one or more quantities of buffered silica gel 30 contained in the plural canister 60 will begin a color change. By noting the number and particular ones of the canisters 60 experiencing this color change, the user can then obtain an approximate determination of his particular blood alcohol concentration ratio.

In Figures 7 through 9, a third embodiment of the breath analyzer device 10 of the present invention is depicted which is constructed in a manner analogous to the first embodiment, but additionally includes internal means for monitoring the volume of breath sample passing through the device. The particular volume monitoring means comprises a piston-like member 70 which is formed to axially reciprocate along the length of the canister 24.

As best shown in Figures 8 and 9, the canister 24 is mounted within the chamber 18 by an end cap 72, the outside diameter of which is equal to the diameter of the chamber 18. The piston-like member 70 includes an axial aperture 76 sized to be slightly greater than the diameter of the canister 24 and an outside diameter sized to be slightly less than the diameter of the chamber 18. As such, the piston-like member 70 is free to reciprocate axially along the length of the canister 24 from the mouth piece end 14 of the housing 12 toward the end plate 72.

Both the piston-like member 70 and end plate 72 include a plurality of apertures 78 and 80, respectively, which are oriented with respect to one another such that their axis are non-aligned. The diameters of the apertures 78 and 80 are sized to be proportional to the central aperture 28 formed within the canister 24 such that as a correlation between the amount of breath sample passing through the apertures 78 and 80 and the central aperture 28 of the canister 24 is achieved.

Thus in use, when an air sample is blown into the housing 12, the breath sample will simultaneously pass through the aperture 78 and 80 and central aperture 28 of the canister 24. However, due to the aperture 78 and 80 metering the amount of air passing therethrough, the piston-like member 70 will simultaneously begin reciprocating from the mouth piece end 14 of the housing 12 toward the end plate 72. This axial reciprocation will continue until such time as the piston-like member 70 abuts the end plate 72 wherein, due to the nonalignment of the apertures 78 and 80, air flow through the apertures 78 and 80 will be discontinued. By proper sizing of the aperture 78 and 80 in proportion to the sizing of the central aperture 28 of the canister 24, the abutment of the piston-like member 70 against the end plate 72 can be timed to represent a certain volume of air passing through the housing 12.

Thus, upon confronting the additional flow restriction encountered when the piston-like member 70 abuts the end plate 72, a user will be alerted to discontinue any further volume of breath sample through the device. With the breath sample discontinued, the user may then view the color change of the silica gel material 30 in a manner previously described in relation to the first embodiment of the present invention and obtain a determination of his particular blood alcohol concentration level.

Although for purposes of description, certain sizes, configurations, and compounds have been defined, those skilled in the art will recognize that modifications to the same can be made without departing from the spirit of the present invention, and such modifications are clearly contemplated herein.

CLAIMS

What is claimed is:

1. A low cost breath analyzer device (10) characterized by:

a tubular housing (12) having a first portion (14) adapted to be placed in the mouth of a user and a second portion (18) defining a chamber in fluid communication with the first portion;

a quantity of colorimetric material (30) disposed within the chamber (30) adapted to change color in response to the presence of alcohol carried by a breath sample blown through the housing (12) into the chamber;

valve means (40) positioned within the chamber (18) to prevent fluid flow through the chamber (18) into the housing (12); and

indicia (50) positioned relative said colorimetric material (30) to indicate the percent ratio of blood alcohol content in the breath sample blown through the housing (12) into the chamber (18).

2. The device of claim 1 further comprising a cannister (24) positioned within the chamber (18) sized to store the quantity of colorimetric material (30) within the chamber (18).

3. The device of claim 2 further comprising means (52) mounted to an end (20) for indicating the volume of the breath sample blown through the housing (12).

4. The device of claim 2 further comprising a piston (70) disposed within the chamber (18) and reciprocally mounted upon the cannister (24) to monitor the volume of air passing through the the housing (12).

5. The device according to any preceding claim wherein the valve means (40) comprises a check valve positioned within said chamber.

6. The device according to any preceding claim wherein the valve means (40) comprises a pair of check valves (40) each positioned on opposite ends of the cannister (24) within the chamber (18).

7. The device of claim 6 wherein said pair of check valves (40) each comprise a flapper diaphragm valve.

8. A device according to any preceding claim wherein the colorimetric material (30) comprises a potassium dichromate impregnated silica gel.

9. The device of claim 9 wherein said colorimetric material (30) additionally includes a sodium bisulfate buffer.

10. The device of claim 1 wherein the chamber (18) contains plurality of cannisters (60), each containing a quantity of potassium dichromate impregnated silica gel buffered with differing quantities of sodium bisulfate to change color in response to differing quantities of alcohol carried by the breath sample blown through the housing (12).

11. A device according to any preceding claim further comprising a removable seal (42, 46) formed on opposite ends of the housing (12).

12. The device according to any preceding claim wherein said housing is formed of a generally transparent material.

1/2    0133326

Fig. 1

Fig. 2

Fig. 3

Fig. 4

*Fig.5*

*Fig. 6*

*Fig. 7*

*Fig.8*

*Fig.9*